# EUROPÄISCHE PATENTANMELDUNG

(11) **EP 1 843 291 A1**
(43) Veröffentlichungstag der Anmeldung: **10.10.2007**
(21) Anmeldenummer: 06007448.1
(22) Anmeldetag: 07.04.2006
(51) Int. Cl.: G06T 7/00, G06T 17/00, A61F 2/00, G06F 19/00, G09B 23/28

(54) **Verfahren und Vorrichtung zur Erstellung von Prothesenschäften**

(71) Anmelder: Gottinger, Ferdinand, 80796 München (DE)
(72) Erfinder: Gottinger, Ferdinand, 80796 München (DE)
(74) Vertreter: Becker Kurig Straus

(57) **Zusammenfassung**

Gemäß der Erfindung wird ein Verfahren zur Erstellung eines Prothesenschafts für einen Extremitäten-Stumpf eines Patienten bereitgestellt. Das Verfahren umfasst Separieren der Anteile von Knochen-, Muskel- und Fettgewebe aus einer dreidimensionalen Darstellung des Stumpfs, Bestimmen einer Ziel-Kompression des Stumpfs auf der Grundlage des Gewichts des Patienten und der Außenfläche des Stumpfs, Bestimmen einer Vielzahl von Querschnitten des Stumpfs, basierend auf der Ziel-Kompression und einer jeweils vorgegebenen Kompressibilität der Anteile von Muskel- und Fettgewebe, und Erstellen eines stetigen dreidimensionalen Modells des Prothesenschafts aus der Vielzahl von Querschnitten. Die entsprechende Vorrichtung der Erfindung umfasst eine Einrichtung, die angepasst ist für ein Separieren der Anteile von Knochen-, Muskel- und Fettgewebe aus einer dreidimensionalen Darstellung des Stumpf, eine Einrichtung, die angepasst ist für ein Bestimmen einer Ziel-Kompression des Stumpfs basierend auf dem Gewicht des Patienten und der Außenfläche des Stumpfs, eine Einrichtung, die angepasst ist für ein Bestimmen einer Vielzahl von Querschnitten des Stumpfs, basierend auf der Ziel-Kompression und einer jeweils vorgegebenen Kompressibilität der Anteile von Muskel- und Fettgewebe, und eine Einrichtung, die angepasst ist für ein Erstellen eines stetigen dreidimensionalen Modells des Prothesenschafts aus der Vielzahl von Querschnitten.

## Beschreibung

Die vorliegende Erfindung betrifft ein Verfahren und eine Vorrichtung zur Erstellung von Schäften für Prothesen oberer und unterer Extremitäten des Menschen.

Durch die Amputation beispielsweise eines Beines fehlt dem Betroffenen zum Gehen und Stehen der Unterstützungspunkt. Es besteht daher die Aufgabe, eine Prothese für die fehlende Extremität mit dem verbleibenden Stumpf kraftschlüssig zu verbinden. Dies geschieht mittels eines Schaftes, der das Bindeglied zwischen Prothese und Stumpf bildet. Die Prothese nimmt über den Schaft das Körpergewicht und die Kräfte auf, die beim Gehen entstehen. Die Formgebung des Schafts soll verhindern, dass es beim einbeinigen Stand zum Abkippen der Prothese nach außen kommt. Die aktive und nicht mehr aktive Muskulatur umlagert den Knochen, so dass eine stabile Verbindung zwischen Prothese und Stumpf fehlt. Eine mehr oder weniger große Pseudatrose entsteht. Da Muskel, die am Oberschenkel entspringen und den Unterschenkel bewegen, durch die Amputation ohne Ansatz sind, verlieren sie Muskelmasse und lagern Fett ein. Bei der Gestaltung der Schaftform muss der Zustand berücksichtigt werden.

Bei der herkömmlichen Herstellung von Prothesenschäften für Prothesen von oberen und unteren Extremitäten eines Menschen, beispielsweise von Unterschenkelprothesen, wird in der Regel ein Gipsverfahren angewandt. Die Form des Stumpfes wird mittels eines Gipsabdrucks abgenommen, und eine Positivform wird daraus erstellt. Es werden Maße ermittelt, und die Positivform wird anhand der Maße verändert. Diese Veränderung erfolgt manuell, d.h. ausschließlich nach der Erfahrung des entsprechenden Technikers, gegebenenfalls unter Hinzuziehung von empfohlenen Richtlinien. Die endgültige Form des Schaftes wird dann über eine Vielzahl von Anproben und entsprechenden Korrekturen ermittelt. Dies ist ersichtlich sehr aufwendig. Ein Nachweis über die medizinisch richtige und/oder verträglichste Form wird dabei nicht erbracht, d.h. das Vorgehen und die Ergebnisse sind nicht objektiv nachvollziehbar.

Neben dem Gipsverfahren werden auch Oberflächen-Abtastverfahren eingesetzt, beispielsweise mittels eines Oberflächen-Scanners, Magnet- oder Laserscanner oder es werden auf andere Art die äußeren Maße genommen. Die Weiterverarbeitung erfolgt computergestützt, es werden den Maßen entsprechend bestimmte Vorlagen ("templates") von einem Bediener ausgewählt und passend umgeformt. Die Art der Umformung entspricht im Prinzip der Methode der Gipstechnik unter Verwendung von Computern, und ist somit ebenfalls rein subjektiver Art.

Bei der Betrachtung eines Amputierten wird herkömmlicherweise nur das äußere Bild herangezogen. Über den Zustand der Muskulatur und die Lage der Knochen kann nur durch Tasten von außen eine gewisse Vorstellung gewonnen werden. Auch das Volumen und die mögliche Kompression zur Stabilisierung können nur durch Erfahrung eingeschätzt werden. Selbst das Abnehmen von Körpermaßen in genau definierten Höhen und Bereichen ist eine unsichere und wenig nachvollziehbare Methode. Die Genauigkeit bewegt sich im cm-Bereich, ist also nicht besonders hoch.

Der Prothesenschaft soll so gestaltet sein, dass der Stumpf das Körpergewicht aufnehmen kann. Weiterhin soll die Prothese einwandfrei gesteuert werden können. Eine Veränderung der Wirbelsäule durch Fehlbelastung des Beckens soll nicht verursacht werden. Die venöse und arterielle Durchblutung darf durch den Prothesenschaft nicht behindert werden. Die maximale Flächenbelastung der Stumpfoberfläche soll, dem Krankheitsbild entsprechend, einen medizinisch geforderten Wert pro cm² nicht überschreiten.

Um die einleitende Kraft und das Körpergewicht möglichst gleichmäßig auf den gesamten Stumpf zu verteilen, muss im Schaft von oben nach unten ein Druckgefälle entstehen. Dies wird durch nach unten hin abfallende Kompression erreicht. Da verschiedene Gewebe-Arten im Stumpf jedoch jeweils unterschiedliche Kompressionen zulassen, ist es erforderlich, über die Verteilung der verschiedenen Gewebe Bescheid zu wissen. Dies ist durch rein äußerliche Abtastung/Bildgebung nicht möglich.

Es ist daher die Aufgabe der vorliegenden Erfindung, ein Verfahren und eine entsprechende Vorrichtung sowie ein Computerprogrammprodukt bereitzustellen, welche die Erstellung von Schäften für Prothesen der oberen und unteren Extremitäten des Menschen auf eine objektive Art, basierend auf direkt gewonnenen Messdaten ermöglichen. Die Erfindung ermöglicht die Herstellung eines gut angepassten Prothesenschafts, wobei jedoch die rein subjektiven und manuell durchzuführenden kostenintensiven Arbeiten bei den Verfahren des Stands der Technik vermieden werden können.

Gemäß einem Aspekt der vorliegenden Erfindung wird angegeben ein Verfahren zur Erstellung eines Prothesenschafts für einen Extremitäten-Stumpf eines Patienten, umfassend:
- Separieren der Anteile von Knochen-, Muskel- und Fettgewebe aus einer dreidimensionalen Darstellung des Stumpfs;
- Bestimmen einer Ziel-Kompression des Stumpfs auf der Grundlage des Gewichts des Patienten und der Außenfläche des Stumpfs;
- Bestimmen einer Vielzahl von Querschnitten des Stumpfs, basierend auf der Ziel-Kompression und einer jeweils vorgegebenen Kompressibilität der Anteile von Muskel- und Fettgewebe; und
- Erstellen eines stetigen dreidimensionalen Modells des Prothesenschafts aus der Vielzahl von Querschnitten.

Gemäß einer beispielhaften Ausführungsform umfasst das Verfahren weiter ein Abtasten des Stumpfs zur Gewinnung der dreidimensionalen Darstellung.

Gemäß einer beispielhaften Ausführungsform umfasst das Verfahren weiter ein Bestimmen der Winkel des Prothesenschafts basierend auf der Lage des Knochengewebes.

Gemäß einer beispielhaften Ausführungsform wird die Kompressibilität des Anteils des Muskelgewebes mit maximal 8 % vorgegeben. In einer anderen beispielhaften Ausführungsform wird die Kompressibilität des Anteils des Fettgewebes mit maximal 20 % vorgegeben.

Gemäß einer beispielhaften Ausführungsform wird das Bestimmen der Vielzahl von Querschnitten des Stumpfs weiterhin basierend auf einer Kompensationsgewichtung ausgeführt, welche die natürliche Belastung des Stumpfs berücksichtigt.

Gemäß einer beispielhaften Ausführungsform stellt die gewonnene dreidimensionale Darstellung Knochen-, Muskel- und Fettgewebe in unterschiedlichen Graustufen dar, und das Separieren der Anteile von Knochen-, Muskel- und Fettgewebe erfolgt mittels Graustufenseparierung.

Gemäß einer beispielhaften Ausführungsform wird das Abtasten mittels Computertomographie, Magnet-Resonanz-Tomographie oder Ultraschall durchgeführt.

Gemäß einer beispielhaften Ausführungsform umfasst das Abtasten weiterhin ein 3D-Scannen.

Gemäß einer beispielhaften Ausführungsform umfasst das Verfahren weiter:
- Bestimmen der gewünschten Eintrittsebene des Prothesenschafts;
   und das Erstellen des stetigen dreidimensionalen Modells des Prothesenschafts wird basierend auf der Eintrittsebene ausgeführt. Die Eintrittsebene muss sich am Ramus auf der Innenseite und am Femur auf der Außenseite orientieren. Der hintere Randverlauf kann dem Glutäus, und seitlich dem Trochanter angepasst werden. Jede Schaftform hat Ausschnitte, die sich an der Anatomie des Patienten orientieren, wobei erreicht werden soll, dass die Beweglichkeit des Gelenks erhalten bleibt.

Gemäß einer beispielhaften Ausführungsform umfasst das Verfahren weiter:
- Herstellen des Prothesenschafts auf der Basis des stetigen dreidimensionalen Modells mittels eines Formgebungsverfahrens.

Gemäß einem weiteren Aspekt der Erfindung wird ein Computerprogrammprodukt bereitgestellt, umfassend Programmcode, der auf einem computerlesbaren Medium gespeichert ist, um das Verfahren gemäß dem vorhergehend beschriebenen Verfahren auszuführen, wenn das Computerprogrammprodukt auf einem Computer ausgeführt wird.

Gemäß noch einem weiteren Aspekt der Erfindung wird eine Vorrichtung zur Erstellung eines Prothesenschafts für einen Extremitäten-Stumpf eines Patienten bereitgestellt, umfassend:
- eine Einrichtung, die angepasst ist für ein Separieren der Anteile von Knochen-, Muskel- und Fettgewebe aus einer dreidimensionalen Darstellung des Stumpfs;
- eine Einrichtung, die angepasst ist für ein Bestimmen einer Ziel-Kompression des Stumpfs basierend auf dem Gewicht des Patienten und der Außenfläche des Stumpfs;
- eine Einrichtung, die angepasst ist für ein Bestimmen einer Vielzahl von Querschnitten des Stumpfs, basierend auf der Ziel-Kompression und einer jeweils vorgegebenen Kompressibilität der Anteile von Muskel- und Fettgewebe; und
- eine Einrichtung, die angepasst ist für ein Erstellen eines stetigen dreidimensionalen Modells des Prothesenschafts aus der Vielzahl von Querschnitten.

Gemäß einer beispielhaften Ausführungsform umfasst die Vorrichtung weiter:
- eine Einrichtung zum Abtasten des Stumpfs zur Gewinnung der dreidimensionalen Darstellung;
wobei die Einrichtung ausgewählt ist aus der Gruppe umfassend:
- einen Computertomograph;
- einen Magnet-Resonanz-Tomograph; und
- eine Ultraschallabtasteinrichtung.

Gemäß einer beispielhaften Ausführungsform umfasst die Vorrichtung weiter:
- eine Einrichtung, die angepasst ist für ein Bestimmen der Winkel des Prothesenschafts, basierend auf der Lage des Knochengewebes.

Gemäß einer beispielhaften Ausführungsform ist die Einrichtung für das Bestimmen der Vielzahl von Querschnitten eingerichtet, das Bestimmen weiterhin basierend auf einer Kompensationsgewichtung auszuführen, welche die natürliche Belastung des Stumpfs berücksichtigt.

Gemäß einer beispielhaften Ausführungsform ist die Einrichtung zum Abtasten des Stumpfs eingerichtet, die dreidimensionale Darstellung Knochen-, Muskel- und Fettgewebe in unterschiedlichen Graustufen darzustellen; und die Einrichtung zum Separieren der Anteile von Knochen-, Muskel- und Fettgewebe ist eingerichtet, die Separierung mittels Graustufenseparierung auszuführen.

Gemäß einer beispielhaften Ausführungsform umfasst die Vorrichtung weiter:
- einen 3D-Scanner, eingerichtet zum Oberflächen-Abtasten des Stumpfs.

Gemäß einer beispielhaften Ausführungsform umfasst die Vorrichtung weiter:
- eine Einrichtung, die eingerichtet ist zum Bestimmen der gewünschten Eintrittsebene des Prothesenschafts;
wobei die Einrichtung zum Erstellen des stetigen dreidimensionalen Modells des Prothesenschafts eingerichtet ist, das Erstellen basierend auf der Eintrittsebene auszuführen.

Gemäß einer beispielhaften Ausführungsform umfasst die Vorrichtung weiter:
- eine Formgebungs-Vorrichtung, die eingerichtet ist zum Herstellen des Prothesenschafts auf der Basis des stetigen dreidimensionalen Modells.

Gemäß einer beispielhaften Ausführungsform ist die Formgebungs-Vorrichtung eine CNC-Fräsvorrichtung oder eine Rapid-Prototyping-Vorrichtung.

### Kurze Beschreibung der Zeichnung

Fig. 1a bzw. 1b zeigen schematisch die Kraftverhältnisse in einem Prothesenschaft ohne Kompression bzw. mit einer gewünschten Kompression;
Fig. 2a und 2b zeigen in einer schematischen Querschnittsansicht, wie erfindungsgemäß die unterschiedliche Kompressibilität verschiedener Gewebearten berücksichtigt wird, um den Ziel-Außenumfang des Prothesenschafts zu bestimmen; und
Fig. 3 ist eine dreidimensionale Ansicht eines fertigen Prothesenschafts, der aus Querschnitten gewonnen wurde, wie sie in Fig. 2 gezeigt werden.

### Detaillierte Beschreibung der Erfindung

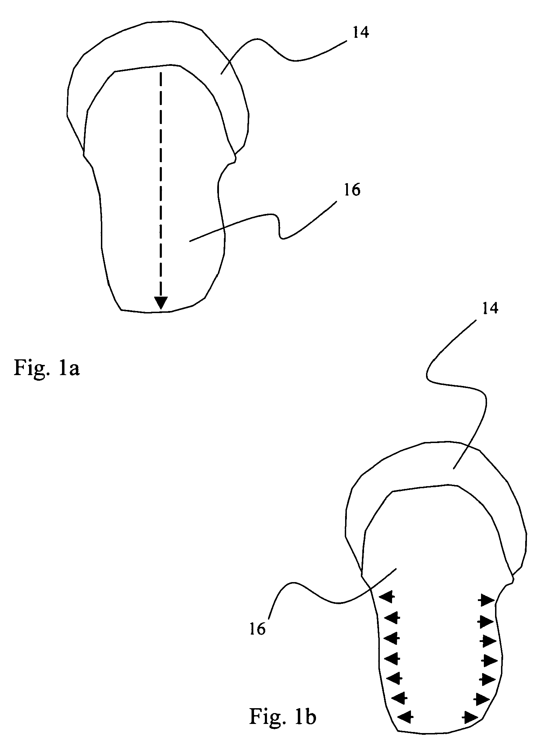

Fig. 1a und 1b sind eine schematische Darstellung der Situation bei einer Unterschenkelprothese bzw. deren zugehörigen Schaft. Gezeigt ist der Oberschenkelstumpf 14, an dem der Prothesenschaft 16 sitzt. Eine Situation ohne Kompression des Stumpfes würde dazu führen, dass die Belastung einseitig nur auf das untere Ende des Prothesenschaftes bzw. Stumpfende wirkt, wie in der Figur 1a gezeigt (gestrichelter Pfeil). Dies ist medizinisch unmöglich, und würde außerdem dazu führen, dass die Prothese instabil und kaum steuerbar wird.

Die erwünschte Druckverteilung muss daher wie in der Figur 1b gezeigt beschaffen sein. Um die einleitende Kraft und das Körpergewicht möglichst gleichmäßig auf den gesamten Stumpf zu verteilen, muss im Schaft von oben nach unten ein Druckgefälle entstehen. Dies wird durch nach unten hin abfallende Kompression des Stumpfes erreicht. Dazu muss das den Oberschenkelknochen umschließende Gewebe verdrängt und verdichtet werden. Um diese gewünschte Kompressionsverteilung zu erreichen, angedeutet durch die Pfeile, ist es erforderlich, über die Verteilung der verschiedenen Gewebe Bescheid zu wissen. Dies liegt darin begründet, dass verschiedene Gewebe-Arten im Stumpf jeweils unterschiedliche Kompressionen zulassen. Knochengewebe sind im Wesentlichen inkompressibel, wohingegen Fettgewebe stärker komprimierbar ist, und Muskelgewebe in der Kompressibilität dazwischen liegt.

Nach den herkömmlichen Verfahren ist es jedoch nicht möglich, die Verteilung bzw. Anordnung dieser Gewebe innerhalb des Stumpfes in einer objektiven Art und Weise zu bestimmen. Aus einer rein äußerlichen Bildgebung, beispielsweise durch Oberflächen-Scanner, manuelles Gewinnen von Maßen und dergleichen, sind diese Daten nicht ableitbar. Herkömmlicherweise wird durch Abtasten des Stumpfs ein grobes Bild gewonnen, im Wesentlichen beruht die nötige Anpassung des Schaftes an die individuellen Gegebenheiten des Stumpfes jedoch auf rein subjektiver Erfahrung und persönlichem Können des entsprechenden Technikers. Dadurch ist eine sichere Versorgung mit gleich bleibender Qualität unmöglich. Auch können die vorgenommenen Änderungen/Anpassungen nicht in einer objektiven Weise nachvollzogen und gegebenenfalls nachgeprüft werden. Diese herkömmliche Erstellungsweise verursacht durch den hohen Anteil manueller Arbeit hohe Kosten und kann eine gute Versorgung nicht sicherstellen.

Die Erfindung hat daher die Aufgabe, diese herkömmlichen Verfahren zu ersetzen, wobei geringere Kosten mit verbesserter Versorgung sowie objektiver Nachprüfbarkeit verbunden werden können.

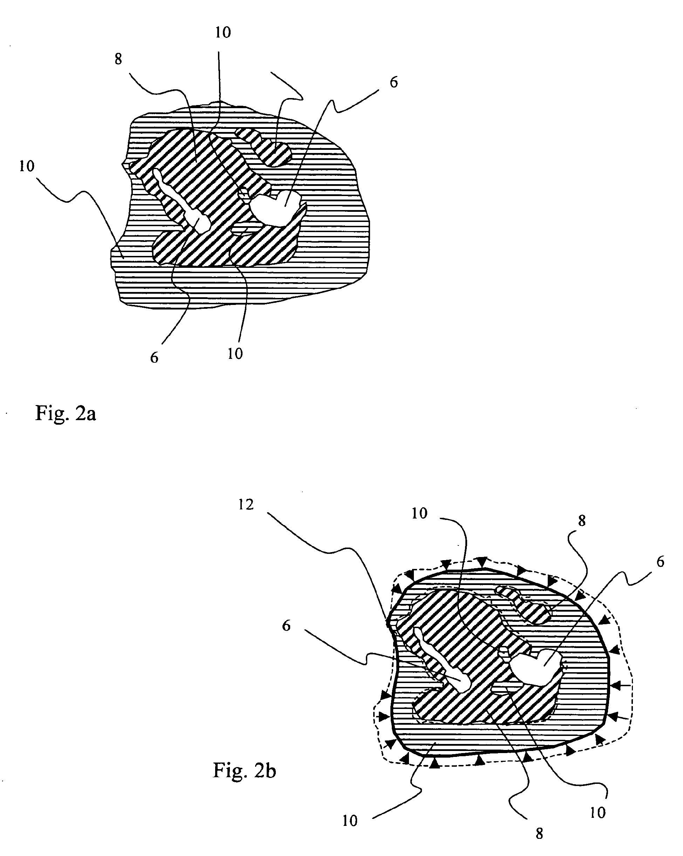

In Figur 2a und 2b ist jeweils beispielhaft ein Querschnitt durch einen Amputationsstumpf gezeigt. Eine solche Querschnittsaufnahme kann durch bekannte Verfahren wie Computertomographie, Magnet-Resonanz-Tomographie, Ultraschall etc. gewonnen werden. Entscheidend ist, dass mit dem verwendeten Bildgebungsverfahren zumindest die drei Gewebetypen Knochen-, Muskel- und Fettgewebe in einer Weise dargestellt werden können, so dass eine Separierung ermöglicht wird. In dem gezeigten Querschnitt sind zwei Bereiche von Knochengewebe 6, zwei Bereiche mit Muskelgewebe 8, sowie drei Bereiche von Fettgewebe 10 dargestellt. In Figur 2a ist eine Situation ohne angelegten Prothesenschaft gezeigt. Die Gewebe sind hier nicht komprimiert.

In Figur 2b ist dagegen die Situation mit angelegtem Schaft bzw. die gewünschte Situation mit optimaler Kompression dargestellt. Wie durch die gestrichelten Linien und Pfeile angezeigt, wird das Fettgewebe 10 am stärksten komprimiert, beispielsweise um einen Faktor von 20 %, bezogen auf die Fläche (Zeichnung nicht maßstabsgetreu). Das Muskelgewebe 8 wird dagegen deutlich weniger komprimiert, wie im Vergleich mit dem gestrichelt dargestellten unkomprimierten Zustand ersichtlich ist. Eine Kompression des Knochengewebes 6 wird vernachlässigt.

Die unter Berücksichtung der Kompressibilität der Gewebetypen gewonnene Kontur bzw. der Außenumfang 12 kann nun dazu verwendet werden, den Prothesenschaft zu erstellen. Dazu wird das hier schematisch gezeigte Verfahren der Flächenreduktion von Muskel- und Fettgewebe über den gesamten Stumpf durchgeführt, beispielsweise mit Querschnitten im Abstand von 2-4 mm oder dergleichen. Aus den so erhaltenen Querschnitten 12 kann dann eine stetige dreidimensionale Form bzw. ein Modell erstellt werden, auf dessen Grundlage der endgültige Prothesenschaft hergestellt wird.

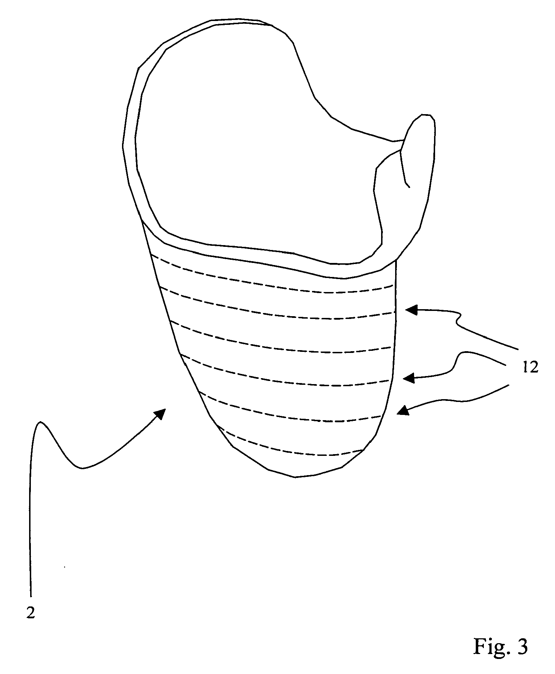

Ein solcher fertiger Prothesenschaft 2 ist in Fig. 3 dargestellt. Hier sind mit gestrichelten Linien beispielhaft (und nicht maßstabsgetreu) die Querschnitte 12 angedeutet, welche gemäß der Fig. 2a bzw. 2b erhalten werden können. In Realität werden diese Querschnitte natürlich deutlich enger angeordnet sein, und es werden auch wesentlich mehr davon verwendet.

Um alle für die Erstellung eines Schaftes gemäß der Erfindung notwendigen Daten zu erhalten, werden CT-, MRT- oder Ultraschall und 3D-Scannerdaten des zu versorgenden Patienten erhoben. Mittels solcher Bildgebungswerkzeuge werden millimetergenaue Umfangs-, Längen- und Winkelmaße gemessen. Durch Einbettung von einzelnen Schichtaufnahmen in verschiedenen Ebenen eines 3D-Modells kann der Schaft entsprechend den Stumpfgegebenheiten modelliert werden.

Aufgrund der unterschiedlichen Kompressibilität verschiedener Gewebetypen ist es dabei wichtig, über den Anteil an Fett- und Muskelgewebe Bescheid zu wissen. Wichtig ist dabei, wie stark das Fett- und Muskelgewebe an welcher Stelle komprimiert werden darf, wo der Stumpf zu entlasten ist und wo er maximal belastet werden kann. Aus dieser Kenntnis lässt sich die Kompression in den verschiedenen Regionen berechnen. Da neben der richtigen Kompression auch die Führung der Prothese und die effektive Lastübernahme wichtig sind, muss ein möglichst enger Kontakt zum Femur und Ramus gewährleistet sein. Aus einer Computertomographie oder Magnet-Resonanz-Tomographie vom Stumpf lassen sich mit der Erfindung die knöchernen Anteile separieren. Unter Berücksichtigung einer vorzugebenden (etwa medizinisch verträglichen) Kompression der ebenfalls separierten Anteile von Muskel- und Fettgewebe kann so die Formung des Schaftes optimal gestaltet werden.

Mit einer speziellen Software werden aus den Schichtaufnahmen 3D-Modelle erstellt, wobei Muskeln, Knochen und Fettgewebe beispielsweise in unterschiedlichen Graustufen abgebildet werden. Anhand einer Graustufenseparierung werden nun die einzelnen Gewebeanteile volumenmäßig erfasst. Die knöchernen Bestandteile geben die Winkel und Steuerungspunkte des Prothesenschafts vor. Anhand der Berechnung des notwendigen inneren Drucks des Schaftes, der sich aus Körpergewicht und Stumpfoberfläche ergibt, wird nun die (flächenbezogene spezifische) Kompression des Fett- und Muskelgewebes berechnet und den Stumpfregionen zugeordnet.

So kann ein Modell des Prothesenschafts gewonnen werden, indem unter Berücksichtigung der gewünschten Kompressionsverhältnisse im Schaft der Ziel-Umfang des Prothesenschafts für eine Vielzahl von Querschnitten bestimmt wird. Aus den Querschnitten wird dann wiederum ein stetiges 3D-Modell erstellt. Dieses Modell kann dann die Grundlage der eigentlichen Herstellung des Prothesenschafts bilden. Damit lassen sich auf CAD-bzw. CNC-Fräsen Modelle erstellen; ebenso ist eine Konstruktion in Rapid Prototyping bzw. 3D-Printing-Verfahren möglich.

Dieses Modell kann also umgestaltet werden, dass daraus ein Prothesenschaft wird, der den gestellten Anforderungen gerecht wird. Es kann beispielsweise vorgegeben werden, dass sich Fettgewebe um ca. 20% und Muskelgewebe um 8% reduzieren lässt. Das Körpergewicht, das sich beim Gehen mit der Ganggeschwindigkeit vervielfacht, muss gleichmäßig auf den Schaft übertragen werden. Dazu muss die benutzbare Stumpfoberfläche berechnet werden.

In einem Beispiel wäre etwa der Umfang oben 55 cm, unten 40 cm, die Stumpflänge betrüge 30 cm, und das Gewicht 80 kg. In diesem Fall ergäbe sich eine Fläche von ca. 1500 cm², und bei im Wesentlichen einbeiniger Belastung ein Druck pro Fläche von etwa 40 g pro cm². Diese Zahlenwerte sind lediglich als beispielhaft zu verstehen. Je nach Ganggeschwindigkeit vervielfacht sich diese Belastung. Sie sollte beispielsweise einen Druck von ca. 100 g pro cm² bei Dauerbelastung und ca. 350 g pro cm² bei kurzzeitiger Belastung (etwa beim Gehen) nicht überschreiten.

Zu bestimmen ist, welche Kompression auf den Stumpf auszuüben ist, um eine Lastübernahme zu erreichen. Ohne Kompression, d.h. seitlich kraftschlüssige Verbindung mit dem Prothesenschaft, würde das gesamte Körpergewicht auf das Stumpfende übertragen werden. Das ist medizinisch nicht möglich, außerdem würde die Prothese instabil werden.

Es ist daher die Aufgabe, eine erwünschte Druckverteilung zu bestimmen. Der Muskel- und Fettanteil bestimmt nicht nur die Verminderung des Schaftvolumens gegenüber der reinen äußerlichen Form im unbelasteten Zustand, sondern auch dessen Formgebung. Die Form muss so gestaltet sein, dass die Muskelaktivität erhalten bleibt. Um eine gute Führung zu erreichen, muss der Ramus umfasst werden und die laterale Anlage an der richtigen Stelle sitzen.

Aus der äußeren Form des Stumpfes, d.h. den gewonnenen Querschnitten, wird eine geschlossene stetige Form generiert, beispielsweise als STL-Datensatz. Diese Form wird nun nach dem Ergebnis des notwendigen Druckaufbaus bzw. der gewünschten Kompression unter Berücksichtigung der anatomischen Stumpfform in ein Funktionsmodell ummodelliert. Für Bereiche, die sich nicht eindeutig berechnen lassen, z.B. die Schaftrandgestaltung der Eintrittsebene, werden Standardformen angepasst. Das so konstruierte 3D-Modell kann nun virtuell über das Stumpfmodell geschoben und die Veränderung des Stumpfes, Gewebekompression, gleichmäßiger Kontakt, Führung durch die knöchernen Anteile des Stumpfes auf seine richtige Lage der Anstützung hin, sowie die Formgebung des Schaftes können abschließend kontrolliert werden.

Die Erfindung ermöglicht gegenüber den herkömmlichen Verfahren des Stands der Technik, auf eine objektive Art und Weise Schäfte für Prothesen zu erstellen. Dies gewährleistet eine bessere Versorgung durch erhöhte Passform, verringert die Herstellungskosten deutlich durch den Entfall kostenintensiver manueller Nacharbeitung und Anpassung, und kann auch die Patientenversorgung beschleunigen, da die auch zeitaufwendigen Anpassungsschritte zumindest in der Anzahl stark verringert werden können oder sogar ganz entfallen. Durch das erfindungsgemäße Verfahren wird ferner eine Nachvollziehbarkeit erreicht, da die Erstellung des Prothesenschafts auf überprüfbaren Grundlagen stattfindet.

## Patentansprüche

1. Verfahren zur Erstellung eines Prothesenschafts für einen Extremitäten-Stumpf eines Patienten, umfassend:
- Separieren der Anteile von Knochen-, Muskel- und Fettgewebe aus einer dreidimensionalen Darstellung des Stumpfs;
- Bestimmen einer Ziel-Kompression des Stumpfs auf der Grundlage des Gewichts des Patienten und der Außenfläche des Stumpfs;
- Bestimmen einer Vielzahl von Querschnitten des Stumpfs, basierend auf der Ziel-Kompression und einer jeweils vorgegebenen Kompressibilität der Anteile von Muskel- und Fettgewebe; und
- Erstellen eines stetigen dreidimensionalen Modells des Prothesenschafts aus der Vielzahl von Querschnitten.

2. Verfahren nach Anspruch 1, weiter umfassend:
- Abtasten des Stumpfs zur Gewinnung der dreidimensionalen Darstellung.

3. Verfahren nach Anspruch 1 oder 2, weiter umfassend:
- Bestimmen der Winkel des Prothesenschafts basierend auf der Lage des Knochengewebes.

4. Verfahren nach einem der vorhergehenden Ansprüche, wobei die Kompressibilität des Anteils des Muskelgewebes mit maximal 8 % vorgegeben wird.

5. Verfahren nach einem der vorhergehenden Ansprüche, wobei die Kompressibilität des Anteils des Fettgewebes mit maximal 20 % vorgegeben wird.

6. Verfahren nach einem der vorhergehenden Ansprüche, wobei das Bestimmen der Vielzahl von Querschnitten des Stumpfs weiterhin basierend auf einer Kompensationsgewichtung ausgeführt wird, welche die natürliche Belastung des Stumpfs berücksichtigt.

7. Verfahren nach einem der vorhergehenden Ansprüche, wobei die gewonnene dreidimensionale Darstellung Knochen-, Muskel- und Fettgewebe in unterschiedlichen Graustufen darstellt, und das Separieren der Anteile von Knochen-, Muskel- und Fettgewebe mittels Graustufenseparierung erfolgt.

8. Verfahren nach einem der vorhergehenden Ansprüche, wobei das Abtasten mittels Computertomographie durchgeführt wird.

9. Verfahren nach einem der Ansprüche 1 bis 7, wobei das Abtasten mittels Magnet-Resonanz-Tomographie durchgeführt wird.

10. Verfahren nach einem der Ansprüche 1 bis 7, wobei das Abtasten mittels Ultraschall durchgeführt wird.

11. Verfahren nach einem der Ansprüche 8 bis 10, wobei das Abtasten weiterhin ein 3D-Scannen umfasst.

12. Verfahren nach einem der vorhergehenden Ansprüche, weiter umfassend:
- Bestimmen der gewünschten Eintrittsebene des Prothesenschafts;
wobei das Erstellen des stetigen dreidimensionalen Modells des Prothesenschafts basierend auf der Eintrittsebene ausgeführt wird.

13. Verfahren nach einem der vorhergehenden Ansprüche, weiter umfassend:
- Herstellen des Prothesenschafts auf der Basis des stetigen dreidimensionalen Modells mittels eines Formgebungsverfahrens.

14. Computerprogrammprodukt, umfassend Programmcode, der auf einem computerlesbaren Medium gespeichert ist, um das Verfahren nach einem der vorhergehenden Ansprüche auszuführen, wenn das Computerprogrammprodukt auf einem Computer ausgeführt wird.

15. Vorrichtung zur Erstellung eines Prothesenschafts für einen Extremitäten-Stumpf eines Patienten, umfassend:
- eine Einrichtung, die angepasst ist für ein Separieren der Anteile von Knochen-, Muskel- und Fettgewebe aus einer dreidimensionalen Darstellung des Stumpfs;
- eine Einrichtung, die angepasst ist für ein Bestimmen einer Ziel-Kompression des Stumpfs basierend auf dem Gewicht des Patienten und der Außenfläche des Stumpfs;
- eine Einrichtung, die angepasst ist für ein Bestimmen einer Vielzahl von Querschnitten des Stumpfs, basierend auf der Ziel-Kompression und einer jeweils vorgegebenen Kompressibilität der Anteile von Muskel- und Fettgewebe; und
- eine Einrichtung, die angepasst ist für ein Erstellen eines stetigen dreidimensionalen Modells des Prothesenschafts aus der Vielzahl von Querschnitten.

16. Vorrichtung nach Anspruch 15, weiter umfassend:
- eine Einrichtung zum Abtasten des Stumpfs zur Gewinnung der dreidimensionalen Darstellung;
wobei die Einrichtung ausgewählt ist aus der Gruppe umfassend:
- einen Computertomograph;
- einen Magnet-Resonanz-Tomograph; und
- eine Ultraschallabtasteinrichtung.

17. Vorrichtung nach Anspruch 15 oder 16, weiter umfassend:
- eine Einrichtung, die angepasst ist für ein Bestimmen der Winkel des Prothesenschafts, basierend auf der Lage des Knochengewebes.

18. Vorrichtung nach einem der Ansprüche 15 bis 17, wobei die Einrichtung für das Bestimmen der Vielzahl von Querschnitten eingerichtet ist, das Bestimmen weiterhin basierend auf einer Kompensationsgewichtung auszuführen, welche die natürliche Belastung des Stumpfs berücksichtigt.

19. Vorrichtung nach einem der Ansprüche 15 bis 18, wobei
- die Einrichtung zum Abtasten des Stumpfs eingerichtet ist, die dreidimensionale Darstellung Knochen-, Muskel- und Fettgewebe in unterschiedlichen Graustufen darzustellen; und
- die Einrichtung zum Separieren der Anteile von Knochen-, Muskel- und Fettgewebe eingerichtet ist, die Separierung mittels Graustufenseparierung auszuführen.

20. Vorrichtung nach einem der Ansprüche 15 bis 19, weiter umfassend:
- einen 3D-Scanner, eingerichtet zum Oberflächen-Abtasten des Stumpfs.

21. Vorrichtung nach einem der Ansprüche 15 bis 20, weiter umfassend:
- eine Einrichtung, die eingerichtet ist zum Bestimmen der gewünschten Eintrittsebene des Prothesenschafts;
wobei die Einrichtung zum Erstellen des stetigen dreidimensionalen Modells des Prothesenschafts eingerichtet ist, das Erstellen basierend auf der Eintrittsebene auszuführen.

22. Vorrichtung nach einem der Ansprüche 15 bis 21, weiter umfassend:
- eine Formgebungs-Vorrichtung, die eingerichtet ist zum Herstellen des Prothesenschafts auf der Basis des stetigen dreidimensionalen Modells.

23. Vorrichtung nach Anspruch 22, wobei die Formgebungs-Vorrichtung eine CNC-Fräsvorrichtung ist.

24. Vorrichtung nach Anspruch 22, wobei die Formgebungs-vorrichtung eine Rapid-Prototyping-Vorrichtung ist.
